# EUROPEAN PATENT APPLICATION

(11) **EP 4 813 972 A1**
(43) Date of publication of application: **30.09.2026**
(21) Application number: 24893457.2
(22) Date of filing: 20.11.2024
(51) Int. Cl.: C07D 211/54, A61K 31/445, A61P 7/02

(54) **CRYSTAL FORM OF BENZENE SULFONATE CONTAINING PIPERIDINE RING COMPOUND, AND PREPARATION METHOD THEREFOR AND USE THEREOF**

(30) Priority: 24.11.2023 CN 202311584011
(71) Applicant: Shanghai Curegene Pharmaceutical Co., Ltd., Shanghai 200135 (CN)
(72) Inventor: HE, Gongxin, Shanghai 200135 (CN); HOU, Kai, Shanghai 200135 (CN); TANG, Xiubo, Shanghai 200135 (CN); FAN, Wenyuan, Shanghai 200135 (CN)
(74) Representative: Vossius & Partner Patentanwälte Rechtsanwälte mbB
(86) International application number: PCT/CN2024/133202
(87) International publication number: WO 2025/108300

(57) **Abstract**

Disclosed in the present invention are a crystal form of a benzene sulfonate containing a piperidine ring compound, and a preparation method therefor and the use thereof. Provided in the present invention is a crystal form A1 of compound (2). In an X-ray powder diffraction pattern (Cu-Kα radiation) of the crystal form at diffraction angles as represented by the 2θ angle, diffraction peaks appear at 5.971° ± 0.200 °, 7.088 ° ± 0.200 °, 12.068 ° ±0.200 °, 15.881 ° ± 0.200 °, 20.557 ° + 0.200 ° and 21.599° ± 0.200 °. The crystal form can exist stably, and the content of isomer impurities as represented by formula (1') can be obviously reduced by means of crystal form preparation steps.

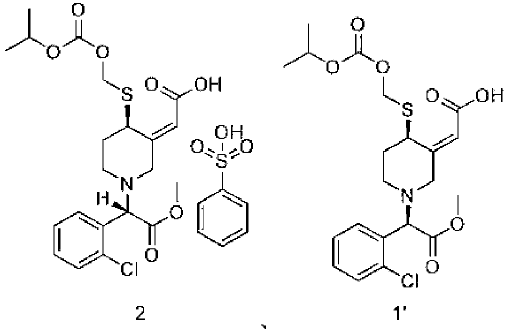

## Description

The present application claims priority to Chinese Patent Application No. CN2023115840114 filed on November 24, 2023, which is incorporated herein by reference in its entirety.

### TECHNICAL FIELD

The present disclosure relates to a crystal form of benzenesulfonate of a piperidine ring-containing compound, a preparation method therefor, and use thereof.

### BACKGROUND

Compound 1 is a compound with anti-platelet activity. The compound can, under the action of a hydrolase *in vivo,* generate an active metabolite of clopidogrel, thereby further irreversibly inhibiting platelet coagulation activity.

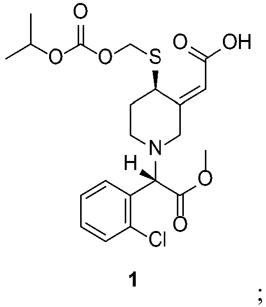

The crude product of compound 1 obtained by the preparation process in the prior art contains an isomer impurity represented by formula 1' (impurity 1' for short). Impurity 1' cannot be removed by conventional purification means such as recrystallization and column chromatography, and compound 1 cannot form a salt with a stable crystal form with a conventional acid or base, such as hydrochloric acid, sulfuric acid, and sodium hydroxide.

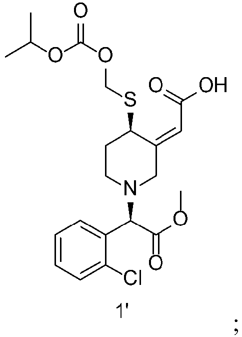

Characterization data for impurity 1'; **LCMS:** [M+ H] ⁺ = 472.1; **¹H NMR:** (400 MHz, DMSO-d6) δ = 12.52 (br s, 1H), 7.53 - 7.44 (m, 2H), 7.43 - 7.34 (m, 2H), 5.66 (s, 1H), 5.34 (br d, J = 2.8 Hz, 1H), 5.27 (d, J = 12.1 Hz, 1H), 5.10 (d, J = 12.1 Hz, 1H), 4.82 - 4.70 (m, 2H), 3.65 (s, 3H), 3.45 (d, J = 12.1 Hz, 1H), 3.10 (br d, J = 12.5 Hz, 1H), 2.75 - 2.57 (m, 2H), 2.11 - 1.98 (m, 1H), 1.82 (br dd, J = 1.8, 14.2 Hz, 1H), 1.22 (dd, J = 2.8, 6.2 Hz, 6H).

### SUMMARY

The technical problem to be solved in the present disclosure is to overcome the defect in the prior art that an isomer impurity represented by formula 1' is difficult to remove, in particular, to reduce the content of impurity 1' from about 2% to a lower level, thereby providing a crystal form of benzenesulfonate of a piperidine ring-containing compound, a preparation method therefor, and use thereof. It has been unexpectedly found in the present disclosure that compound 1 can form a salt with benzenesulfonic acid and further obtain a stable crystal form. By preparing the crystal form, the isomer impurity described above can be removed. Through this step, the content of the isomer impurity represented by formula 1' can be significantly reduced.

The present disclosure mainly solves the technical problem described above through the following technical solutions.

The present disclosure provides crystal form A1 of compound 2, which has diffraction peaks at 2θ angles of 5.971°±0.200°, 7.088°±0.200°, 12.068°±0.200°, 15.881°±0.200°, 20.557°±0.200°, and 21.599°±0.200° in an X-ray powder diffraction pattern using Cu-Kα radiation;

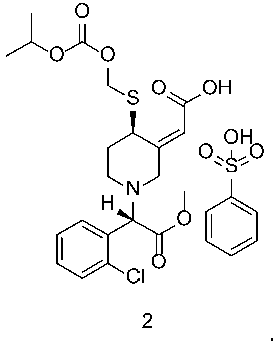

In a certain preferred embodiment, crystal form A1 of compound 2 further has characteristic peaks at one or more 2θ angles of 7.742°±0.200°, 17.257°±0.200°, 21.259°±0.200°, 23.137°±0.200°, and 24.134°±0.200° in an X-ray powder diffraction pattern using Cu-Kα radiation; preferably, diffraction peaks at 2θ angles and relative intensities of crystal form A1 of compound 2 in an X-ray powder diffraction pattern using Cu-Kα radiation are:

| 2θ angle | Relative intensity |
|---|---|
| 5.971° | 100.0% |
| 7.088° | 61.3% |
| 7.742° | 23.7% |
| 10.667° | 5.7% |
| 12.068° | 41.9% |
| 12.357° | 4.7% |
| 13.727° | 6.5% |
| 14.076° | 19.7% |
| 15.881° | 58.6% |
| 17.257° | 22.7% |
| 17.803° | 5.2% |
| 18.184° | 7.0% |
| 18.693° | 4.4% |
| 19.379° | 8.7% |
| 20.225° | 10.8% |
| 20.557° | 42.0% |
| 21.259° | 35.0% |
| 21.599° | 54.1% |
| 22.746° | 18.0% |
| 23.137° | 30.7% |
| 23.657° | 10.3% |
| 24.134° | 29.5% |
| 25.032° | 7.2% |
| 26.215° | 16.0% |
| 27.112° | 2.3% |
| 27.630° | 3.5% |
| 28.106° | 6.2% |
| 29.093° | 4.6% |
| 29.430° | 2.6% |
| 30.103° | 3.8% |
| 32.135° | 2.5% |
| 36.787° | 2.2% |

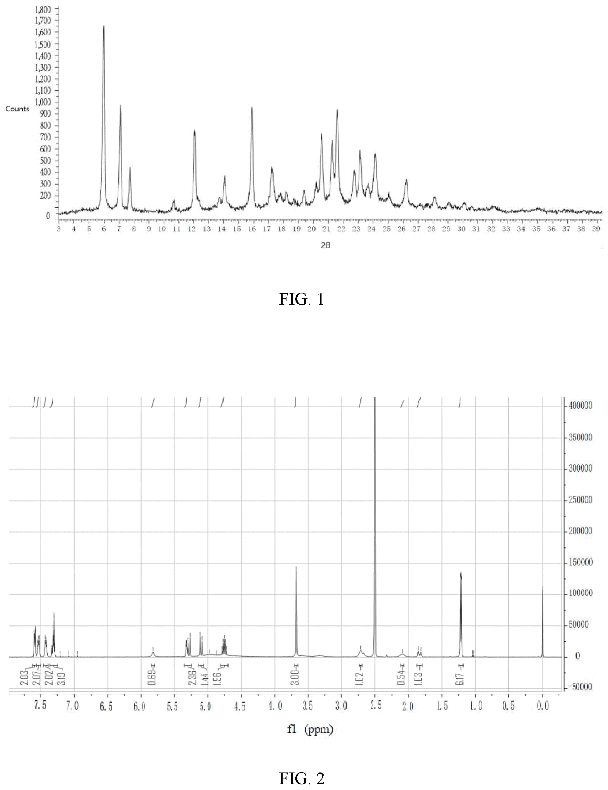

For example, crystal form A1 of compound 2 has an X-ray powder diffraction pattern, expressed in terms of 2θ angles, using Cu-Kα radiation substantially as shown in FIG. 1.

In a certain preferred embodiment, a thermogravimetric analysis (TGA) pattern of crystal form A1 of compound 2 shows a weight loss of 3.63% at 27-100 °C, wherein "%" is mass percentage.

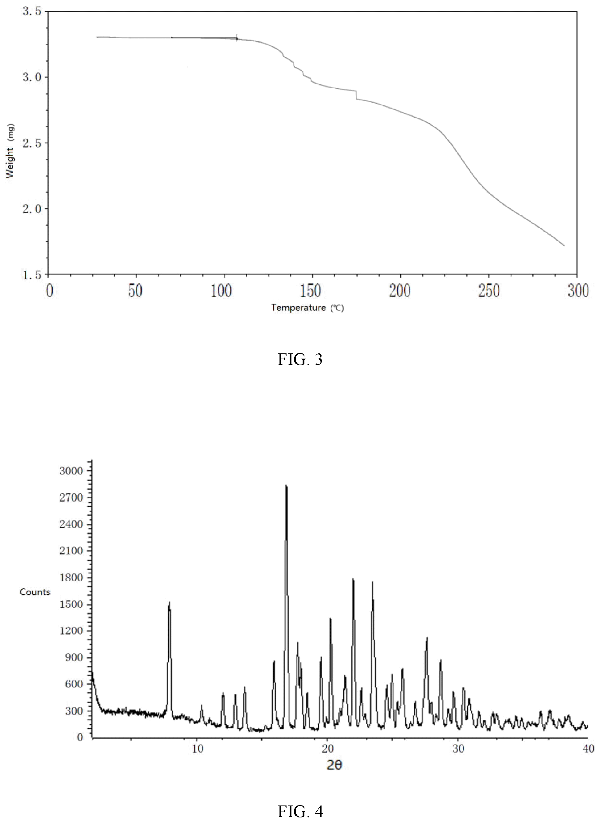

In a certain preferred embodiment, crystal form A1 of compound 2 has a TGA pattern substantially as shown in FIG. 3.

The present disclosure provides crystal form F1 of compound 2, which has diffraction peaks at 2θ angles of 17.006°+0.200°, 20.439°+0.200°, 22.149°+0.200°, and 23.629°+0.200° in an X-ray powder diffraction pattern using Cu-Kα radiation;

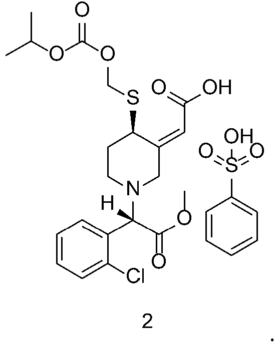

In a certain preferred embodiment, crystal form F1 of compound 2 further has characteristic peaks at one or more 2θ angles of 17.891°±0.200°, 19.671°±0.200°, 27.740°±0.200°, and 28.842°±0.200° in an X-ray powder diffraction pattern using Cu-Kα radiation; preferably, diffraction peaks at 2θ angles and relative intensities of crystal form A1 of compound 2 in an X-ray powder diffraction pattern using Cu-Kα radiation are:

| 2θ angle | Relative intensity |
|---|---|
| 7.978° | 16.10% |
| 10.514° | 3.30% |
| 11.115° | 1.30% |
| 12.164° | 8.90% |
| 13.115° | 11.30% |
| 13.834° | 15.10% |
| 15.404° | 1.50% |
| 16.063° | 29.60% |
| 16.254° | 3.20% |
| 17.006° | 100.00% |
| 17.891° | 41.10% |
| 18.146° | 31.30% |
| 18.617° | 16.70% |
| 19.671° | 34.90% |
| 20.439° | 54.60% |
| 21.186° | 8.80% |
| 21.551° | 29.30% |
| 22.149° | 82.50% |
| 22.741° | 19.70% |
| 23.036° | 7.00% |
| 23.629° | 84.80% |
| 24.692° | 25.60% |
| 25.104° | 30.40% |
| 25.522° | 17.20% |
| 25.915° | 34.10% |
| 26.888° | 14.10% |
| 27.740° | 50.10% |
| 28.118° | 15.30% |
| 28.488° | 8.00% |
| 28.842° | 43.60% |
| 29.426° | 13.80% |
| 29.803° | 22.60% |
| 30.588° | 25.80% |
| 31.022° | 18.40% |
| 31.227° | 12.60% |
| 31.772° | 12.00% |
| 32.153° | 4.40% |
| 32.802° | 11.70% |
| 33.117° | 9.80% |
| 33.785° | 5.60% |
| 34.061° | 6.20% |
| 34.582° | 6.80% |
| 35.040° | 6.40% |
| 35.525° | 5.20% |
| 35.838° | 4.60% |
| 36.460° | 11.30% |
| 37.166° | 13.40% |
| 37.905° | 6.80% |
| 38.351° | 8.30% |
| 38.639° | 9.80% |
| 39.699° | 4.20% |

For example, crystal form F1 of compound 2 has an X-ray powder diffraction pattern, expressed in terms of 2θ angles, using Cu-Kα radiation substantially as shown in FIG. 4.

In a certain preferred embodiment, a thermogravimetric analysis (TGA) pattern of crystal form F1 of compound 2 shows a weight loss of 0.5% at 32-130 °C, wherein "%" is mass percentage.

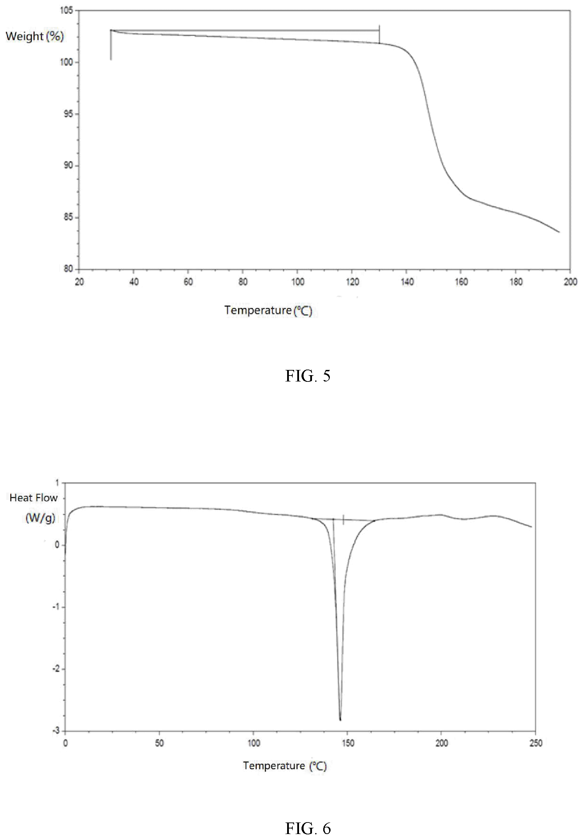

In a certain preferred embodiment, crystal form F1 of compound 2 has a TGA pattern substantially as shown in FIG. 5.

In a certain preferred embodiment, a differential scanning calorimetry (DSC) pattern of crystal form F1 of compound 2 shows an endothermic peak at 147.8 °C.

In a certain preferred embodiment, crystal form F1 of compound 2 has a DSC pattern substantially as shown in FIG. 6.

The present disclosure further provides a preparation method for crystal form A1 of compound 2, which comprises the following step: crystallizing compound 2 in a solvent, or mixing compound 1 with benzenesulfonic acid in a solvent, wherein the solvent is a mixed solvent of an ether solvent and acetonitrile; further, the preparation method comprises the following step: mixing solution 1 containing compound 1 with solution 2 containing benzenesulfonic acid for a reaction, wherein a solvent of solution 1 is a mixed solvent of an ether solvent and acetonitrile; a solvent of solution 2 is a mixed solvent of an ether solvent and/or acetonitrile; the ether solvent may be isopropyl ether;

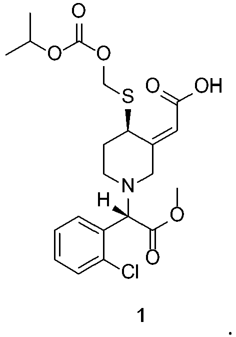

In the preparation method for crystal form A1 of compound 2, a purity of compound 1 may be 90%-99.6%, such as 92.6%, 94.3%, 95.8%, or 99%.

In a certain embodiment, compound 1 is a crude product of compound 1, and the crude product of compound 1 is a mixture of compound 1 and an isomer impurity represented by formula 1'; a mass ratio of compound 1 to the isomer impurity may be (10-500):1, such as 15.433:1, 23.575:1, 23.95:1, or 220:1;

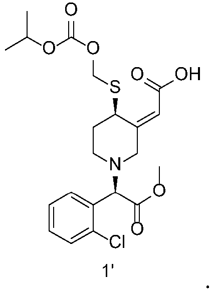

In a certain embodiment, in the preparation method for crystal form A1 of compound 2, when the solvent is an ether solvent and a nitrile solvent, a volume ratio of the ether solvent to the nitrile solvent may be (1-10):1, such as 5:1 or 7:1.

In a certain embodiment, in the preparation method for crystal form A1 of compound 2, a mass-to-volume ratio of compound 1 to the solvent of solution 1 may be 1:(1-15) g/mL, or may be 1:(1-10) g/mL, such as 1:6 g/mL and 1:10 g/mL.

In a certain embodiment, in the preparation method for crystal form A1 of compound 2, a molar ratio of benzenesulfonic acid to compound 1 may be (0.9-1.2):1, such as 1.1:1.

In a certain embodiment, in the preparation method for crystal form A1 of compound 2, a mass-to-volume ratio of benzenesulfonic acid to the solvent of solution 2 may be 1:(0.1-10) g/mL, such as 1:6 g/mL or 1:10 g/mL.

In a certain embodiment, the preparation method for crystal form A1 of compound 2 may further comprise a step of mixing with crystal form A1 of compound 2 described above as a benzenesulfonate seed crystal; a mass ratio of the benzenesulfonate seed crystal to compound 1 may be 1:(20-100), such as 1:50.

In a certain embodiment, in the preparation method for crystal form A1 of compound 2, a mass-to-volume ratio of the benzenesulfonate seed crystal to the solvent of solution 3 may be 1:(6000-10000) g/mL, such as 1:8000 g/mL.

In a certain embodiment, in the preparation method for crystal form A1 of compound 2, solution 2 containing benzenesulfonic acid may be added to solution 1 containing compound 1, and an addition rate may be 0.1-1 V/h, such as 1 V/h.

In a certain embodiment, the preparation method for crystal form A1 of compound 2 may further comprise a step of mixing with solution 3 containing crystal form A1 of compound 2 described above as a benzenesulfonate seed crystal, wherein a solvent of solution 3 is a mixed solvent of an ether solvent and/or acetonitrile; the ether solvent may be isopropyl ether; further, solution 1 containing compound 1 and solution 2 containing benzenesulfonic acid may be added to solution 3 containing the benzenesulfonate seed crystal, and an addition rate may be 0.1-1 V/h, such as 0.4 V/h or 0.8 V/h.

In a certain embodiment, in the preparation method for crystal form A1 of compound 2, a mixing temperature may be a conventional temperature in the art for such reactions, such as 25 °C.

In a certain embodiment, in the preparation method for crystal form A1 of compound 2, a mixing reaction time may be 0.1-100 h, such as 3 h, 17 h, or 72 h.

The present disclosure further provides a preparation method for crystal form F1 of compound 2, which comprises the following step: crystallizing compound 2 in a solvent, or mixing compound 1 with benzenesulfonic acid in a solvent, wherein the solvent is an ester solvent; further, the preparation method comprises the following step: mixing solution 1 containing compound 1 with solution 2 containing benzenesulfonic acid, wherein a solvent of solution 1 is an ester solvent or an ether solvent; a solvent of solution 2 is an ester solvent; the ester solvent may be ethyl acetate and/or isopropyl acetate; the ether solvent may be tetrahydrofuran;

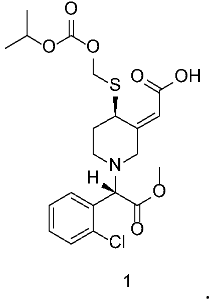

In the preparation method for crystal form F1 of compound 2, a purity of compound 1 may be 90%-99.6%, such as 95.8%, 97.14%, or 97.5%.

In a certain embodiment, compound 1 is a crude product of compound 1, and the crude product of compound 1 is a mixture of compound 1 and an isomer impurity represented by formula 1'; a mass ratio of compound 1 to the isomer impurity may be (10-500):1, such as 23.95:1, 33.965:1, or 42.026:1;

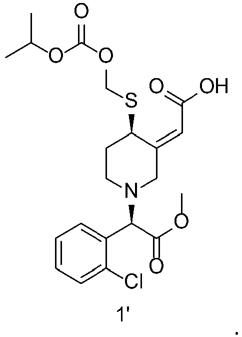

In a certain embodiment, in the preparation method for crystal form F1 of compound 2, a mass-to-volume ratio of compound 1 to the solvent of solution 1 may be 1:(1-15) g/mL, or may be 1:(1-10) g/mL, such as 1:2.3 g/mL, 1:4 g/mL, or 1:6 g/mL.

In a certain embodiment, in the preparation method for crystal form F1 of compound 2, a molar ratio of benzenesulfonic acid to compound 1 may be (0.9-1.2):1, such as 0.98:1 or 1:1.

In a certain embodiment, in the preparation method for crystal form F1 of compound 2, a mass-to-volume ratio of benzenesulfonic acid to the solvent of solution 2 may be 1:(0.1-10) g/mL, such as 1:0.7 g/mL, 1:1 g/mL, or 1:4 g/mL.

In a certain embodiment, the preparation method for crystal form F1 of compound 2 may further comprise a step of mixing with crystal form F1 of compound 2 described above as a benzenesulfonate seed crystal; a molar ratio of compound 1 to the seed crystal is 1:(0.01%-1%), such as 1:0.1%.

In a certain embodiment, the preparation method for crystal form F1 of compound 2 may further comprise a step of mixing with solvent 3, wherein solvent 3 is an alkane solvent, and the alkane solvent may be n-heptane. A mass-to-volume ratio of compound 1 and solvent 3 is 1:(3-45) g/mL, such as 1:27 g/mL.

In a certain embodiment, the preparation method for crystal form F1 of compound 2 may further comprise adding solvent 3 to a mixed solution of solution 1 containing compound 1 and solution 2 containing benzenesulfonic acid, and an addition rate may be 0.1-3 V/h, such as 1.5 V/h.

In a certain embodiment, the preparation method for crystal form F1 of compound 2 may further comprise thermal cycling. The number of thermal cycles may be a conventional number in the art for such reactions, for example, 5. A thermal cycling temperature may be 25 °C to -10 °C.

The present disclosure further provides use of crystal form A1 or F1 of compound 2 described above in the preparation of compound 1;

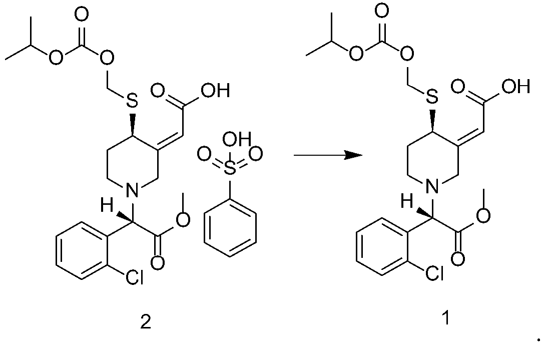

The present disclosure further provides a purification method for compound 1, which comprises the following steps: 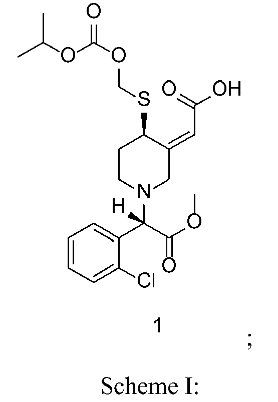
S1: the preparation method for crystal form A1 of compound 2 as described in any one of the preceding embodiments;
S2: subjecting crystal form A1 of compound 2 to a hydrolysis reaction to prepare compound 1;

Scheme II:
S1: the preparation method for crystal form F1 of compound 2 as described in any one of the preceding embodiments;
S2: subjecting crystal form F1 of compound 2 to a hydrolysis reaction to prepare compound 1.

The preferred conditions described above may be combined arbitrarily to obtain preferred embodiments of the present disclosure without departing from the general knowledge in the art.

The reagents and starting materials used in the present disclosure are commercially available.

The positive and progressive effects of the present disclosure are as follows: The present disclosure provides a crystal form of benzenesulfonate of a piperidine ring-containing compound, a preparation method therefor, and use thereof. The crystal form can exist stably, and the content of the isomer impurity represented by formula 1' can be significantly reduced through the steps of preparing the crystal form.

### BRIEF DESCRIPTION OF THE DRAWINGS

FIG. 1 is an XRPD pattern of crystal form A1 of compound 2 in the examples.
FIG. 2 is a ¹H-NMR spectrum of crystal form A1 of compound 2 in the examples.
FIG. 3 is a TGA pattern of crystal form A1 of compound 2 in the examples.
FIG. 4 is an XRPD pattern of crystal form F1 of compound 2 in the examples.
FIG. 5 is a TGA pattern of crystal form F1 of compound 2 in the examples.
FIG. 6 is a DSC pattern of crystal form F1 of compound 2 in the examples.
FIG. 7 is an XRPD pattern of crystal form A2 of compound 2 in the examples.
FIG. 8 is an XRPD pattern of crystal form A3 of compound 2 in the examples.
FIG. 9 is an XRPD pattern of crystal form A4 of compound 2 in the examples.
FIG. 10 is a TGA pattern of crystal form A4 of compound 2 in the examples.
FIG. 11 is a DSC pattern of crystal form A4 of compound 2 in the examples.
FIG. 12 is an XRPD pattern of crystal form A5 of compound 2 in the examples.
FIG. 13 is an XRPD pattern of crystal form B1 of compound 2 in the examples.
FIG. 14 is a TGA pattern of crystal form B1 of compound 2 in the examples.
FIG. 15 is a DSC pattern of crystal form B1 of compound 2 in the examples.
FIG. 16 is an XRPD pattern of crystal form B2 of compound 2 in the examples.
FIG. 17 is an XRPD pattern of crystal form B3 of compound 2 in the examples.
FIG. 18 is an XRPD pattern of crystal form C of compound 2 in the examples.
FIG. 19 is a TGA pattern of crystal form C of compound 2 in the examples.
FIG. 20 is a DSC pattern of crystal form C of compound 2 in the examples.
FIG. 21 is an XRPD pattern of crystal form D of compound 2 in the examples.
FIG. 22 is a TGA pattern of crystal form D of compound 2 in the examples.
FIG. 23 is a DSC pattern of crystal form D of compound 2 in the examples.
FIG. 24 is an XRPD pattern of crystal form E1 of compound 2 in the examples.
FIG. 25 is an XRPD pattern of crystal form E2 of compound 2 in the examples.
FIG. 26 is a TGA pattern of crystal form E2 of compound 2 in the examples.
FIG. 27 is a DSC pattern of crystal form E2 of compound 2 in the examples.
FIG. 28 is an XRPD pattern of crystal form F2 of compound 2 in the examples.
FIG. 29 is an XRPD pattern of crystal form G of compound 2 in the examples.
FIG. 30 is a TGA pattern of crystal form G of compound 2 in the examples.
FIG. 31 is a DSC pattern of crystal form G of compound 2 in the examples.
FIG. 32 is an XRPD pattern of crystal form H of compound 2 in the examples.
FIG. 33 is an XRPD pattern of crystal form I of compound 2 in the examples.
FIG. 34 is a ¹H-NMR spectrum of crystal form F1 of compound 2 in the examples.

### DETAILED DESCRIPTION OF THE PREFERRED EMBODIMENT

The present disclosure is further illustrated by the following examples, which are not intended to limit the present disclosure to the scope of the described examples. Experimental methods without specific conditions indicated in the following examples were performed in accordance with conventional methods and conditions or in accordance with product instructions.

### Comparison table of abbreviations:

| Abbreviation | Chinese name |
|---|---|
| IPE | Isopropyl ether |
| ACN | Acetonitrile |
| EtOH | Ethanol |
| IPA | Isopropyl alcohol |
| MEK | Methyl ethyl ketone |
| MIBK | Methyl isobutyl ketone |
| EA | Ethyl acetate |
| PE | Petroleum ether |
| IPAc | Isopropyl acetate |
| MTBE | Methyl tert-butyl ether |
| MeOH | Methanol |
| DCM | Dichloromethane |
| THF | Tetrahydrofuran |
| IBA | Isobutyl alcohol |
| TFA | Trifluoroacetic acid |

The test methods involved in the following examples were as follows:

### X-Ray powder diffraction(XRPD) method

### Instrument model:PANalytical, Empyrean X-ray powder diffractometer

Test method: The sample was prepared on a zero-background silicon wafer by gently pressing to obtain a flat surface. About 10-20 mg of a sample was used for XRPD analysis.

The detailed XRPD parameters were as follows:

| **Instrument model** | Bruker, D2 Advance |
|---|---|
| **Radiation source** | Cu Kα (λ = 1.5418 Å) |
| **Detector** | LynxEye XE |
| **Scanning angle** | 3-40° (2θ) |
| **Scanning step size** | 0.02° (2θ) |
| **Scanning speed** | 0.200 s/step |
| **Light tube voltage/current** | 30 kV/10 mA |
| **Divergence slit** | 0.6 mm |
| **Rotation** | On |
| **Sample holder** | Zero-background sample tray |

### Thermogravimetric analysis (TGA) method

Instrument model: TA Discovery TGA55 thermogravimetric analyzer
Test method: The sample (3-5 mg) was placed in a pre-weighed aluminum pan and heated according to the parameters shown below, and data analysis was performed using the TRIOS.

The detailed TGA parameters were as follows:

| Sample tray | Aluminum crucible, open |
|---|---|
| Temperature range | 25-300 °C |
| Heating rate | 10 °C/min |
| Purge gas | N₂ |
| Flow rate | Equilibration chamber: 40 mL/min |
| | Sample chamber: 25 mL/min |

### Differential scanning calorimetry (DSC) method

### Instrument model: TA differential scanning calorimeter Discovery DSC 250, United States

Test method: The sample (2-3 mg) was placed in an aluminum pan with pinholes for heating, the heating was performed according to the following parameters, and data analysis was performed using the TRIOS.

The detailed **DSC** parameters were as follows:

| Sample pan | Aluminum crucible with holes |
|---|---|
| Temperature range | 25 - 300 °C |
| Heating rate | 10 °C/min |
| Purge gas | N₂ |
| Flow rate | 50 mL/min |

### DVS:

### Instrument model: SMS, DVS Intrinsic

Test method: 20-30 mg of the sample was placed in an oil-coated sample chamber and weighed automatically. The sample was analyzed according to the set parameters in Table 21.

The detailed **DVS** parameters were as follows:

| dm/dt | 0.002%/min |
|---|---|
| Measurement temperature | 25 °C |
| Minimum dm/dt stability duration | 30 min |
| Maximum dm/dt equilibration time | 120 min |
| Data storage interval | 5 s |
| Gas and flow rate | N₂, 200 sccm |
| Method | Adsorption: 0%, 10%, 20%, 30%, 40%, 50%, 60%, 70%, 80%, 90% RH |
| | Desorption: 90%, 80%, 70%, 60%, 50%, 40%, 30%, 20%, 10%, 0% RH |

### ¹H-NMR method

### Instrument model: Bruker 400 MHz instrument

Test method: ¹H-NMR spectra were collected on a Bruker 400 MHz instrument. The sample was prepared in a DMSO-d6 solvent and tested using the following parameters unless otherwise specified. Data were analysed using MestReNova.

The detailed ¹H-NMR parameters were as follows:

| Frequency | 400 MHz |
|---|---|
| Number of scans | 4 |
| Temperature | 295 K |
| Relaxation time | 1 s |

### High performance liquid chromatography (HPLC)

The detailed parameters were as follows:

| **Instrument model** | Agilent 1260 series HPLC |
|---|---|
| **Chromatographic column** | Ascentis^{®} Express 90 Å C18 150 × 4.6 mm, 2.7 µm |
| **Column temperature** | 40 °C |
| **Mobile phase** | A: 0.02% aqueous TFA solution |
| | B: solution of 0.02% TFA in acetonitrile |
| **Flow rate** | 1.0 mL/min |
| **Injection volume** | 5 µL |
| **Detector** | DAD; |
| **Detection wavelength** | 210 nm |
| **Run time** | 50 min |
| **Diluent** | ACN |
| **Gradient (time (min)/B%)** | 0/10%, 3/30%, 28/45%, 40/95%, 45/95%, 45.1/10%, 50/10% |

### Example 1. Preparation of Compound 1

Compound 1 was prepared by referring to the scheme of Example 1 in Patent WO2022022559, and the content of the impurity of prepared compound 1 was about 4%-6%. After compound 1 was purified by column chromatography (EA/PE = 1:10), the content of impurity 1' was reduced to 2.5%-3%, but could not be further reduced.

### Example 2. Preparation of Crystal Form A1

At 25 °C, compound 1 (2 g, purity: 95.8%, with a content of impurity 1' of 4%) was dissolved in 6 V of an IPE/ACN solution at a volume ratio of 5:1 to obtain mixture 1. Benzenesulfonic acid (684 mg) was added to the IPE/ACN mixed solution (6 V) at a volume ratio of 5:1 to obtain mixture 2. Mixture 1 and mixture 2 were mixed and stirred for 3 h. The resulting mixture was filtered, and the filter cake was dried under vacuum at 30 °C to obtain crystal form A1 of benzenesulfonate of compound 1 with a purity of crystal form A1 of 98.54% and a content of impurity 1' of 0.65%. Tests showed that the crystal form was consistent with that in Example 3.

### Example 3. Preparation of Crystal Form A1

At 25 °C, compound 1 (1 g, purity: 99%, with a content of impurity 1' of 0.45%) was dissolved in 10 V of an IPE/ACN solution at a volume ratio of 7:1. A solution of benzenesulfonic acid (335 mg) in IPE (3350 mL, 10 wt%) was added to the solution at a rate of 1 V/h, and the mixture was stirred for 3 days. The resulting mixture was filtered, and the filter cake was dried under vacuum at 30 °C to obtain crystal form A1 of benzenesulfonate of compound 1 with a purity of 99.5% and a content of impurity 1' of less than 0.1%. The crystal form confirmation results are shown in FIGs. 1-3.

### Example 4. Preparation of Crystal Form A1

At 25 °C, compound 1 (2 g, purity: 94.3%, with a content of impurity 1' of 4%) was dissolved in 6 V of an IPE/ACN solution at a volume ratio of 5:1 to obtain mixture 1. Benzenesulfonic acid (684 mg) was added to the IPE/ACN mixed solution (6 V) at a volume ratio of 5:1 to obtain mixture 2. 40 mg of the seed crystal (such as crystal form A1 of benzenesulfonate prepared in Example 3) was added to 320 mL of an IPE/ACN solution at a volume ratio of 5:1 to obtain mixture 3. Mixture 1 and mixture 2 were added to mixture 3 at a rate of 0.4 V/h, and then the mixture was stirred for 3 h. The resulting mixture was filtered, and the filter cake was dried under vacuum at 30 °C to obtain crystal form A1 of benzenesulfonate of compound 1. Tests showed that the crystal form was consistent with that in Example 3, the purity of crystal form A1 was 99.04%, and the content of impurity 1' was 0.35%.

### Example 5. Preparation of Crystal Form A1

At 25 °C, compound 1 (5 g, purity: 92.6%, with a content of impurity 1' of 6%) was dissolved in 6 V of an IPE/ACN solution at a volume ratio of 5:1 to obtain mixture 1. Benzenesulfonic acid (1.7 g) was added to the IPE/ACN mixed solution (6 V, 3000 mL) at a volume ratio of 5:1 to obtain mixture 2. 100 mg of the seed crystal (such as crystal form A1 of benzenesulfonate prepared in Example 3) was added to 800 mL of an IPE/ACN solution at a volume ratio of 5:1 to obtain mixture 3. Mixture 1 and mixture 2 were added to mixture 3 at a rate of 0.8 V/h, and then the mixture was stirred for 17 h. The resulting mixture was filtered, and the filter cake was dried under vacuum at 30 °C to obtain crystal form A1 of benzenesulfonate of compound 1 with a purity of 99.6% and a content of impurity 1' of 0.21%.

### Example 6. Preparation of Crystal Form F1

At 25 °C, compound 1 (1 g, purity: 95.8%, with a content of impurity 1' of 4%) was dissolved in THF (2.3 V). Benzenesulfonic acid (1 eq) was dissolved in THF (0.7 V). The two solutions were then mixed at 25 °C and stirred for 2 h. n-Heptane (27 V) was added to the mixed solution at a temperature of 25 °C and a flow rate of 1.5 V/h, and after the addition was completed, the temperature was maintained for 3 h. The mixture was filtered, and the filter cake was dried. 50 mg of the dried filter cake was dissolved in isopropyl alcohol (6 V) at 50 °C. The resulting solution or thin suspension was filtered through a 0.45 µm syringe membrane filter, and the filtrate was cooled to 5 °C at a rate of 0.1 °C/min. The sample without a precipitate at 5 °C was further cooled to -20 °C. The mixture was filtered to obtain a filter cake, and the filter cake was dried to obtain crystal form F1 of benzenesulfonate.

### Example 7. Preparation of Crystal Form F1

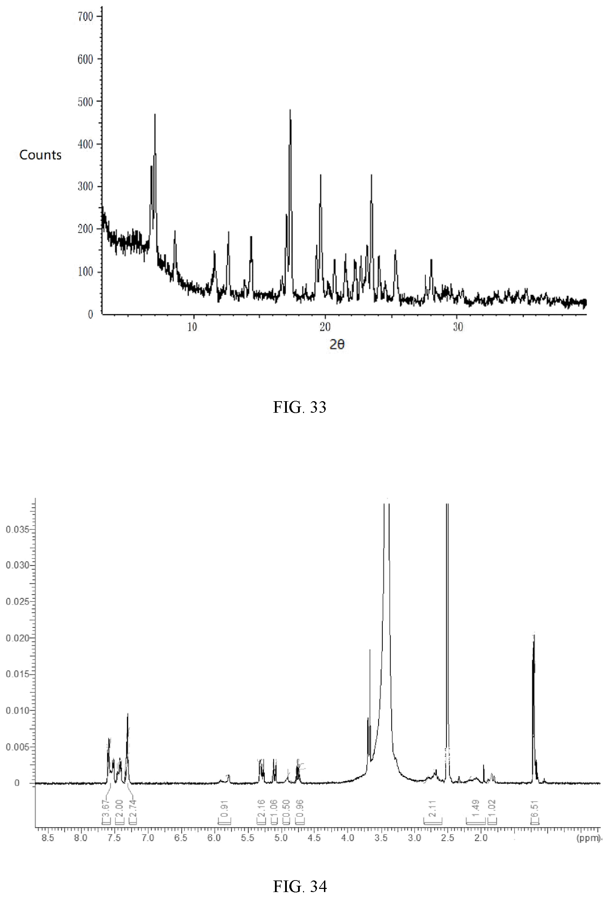

At 25 °C, compound 1 (2 g, purity: 97.14%, with a content of impurity 1' of 2.86%) was added to IPAc (4 V). A solution of benzenesulfonic acid (0.98 eq) in IPAc (1 V) was then added thereto, the mixture was stirred for 20 min, and the seed crystal of crystal form F1 (such as crystal form F1 of benzenesulfonate prepared in Example 6) (0.1% eq) was added. The mixture was then subjected to 5 thermal cycles with a temperature range of 25 °C to -10 °C. After filtration, the filter cake was washed with IPAc (2 V) and dried under a nitrogen flow at 25 °C for 24 h to obtain crystal form F1 of benzenesulfonate of compound 1. The characteristic spectra are shown in FIGs. 4-6 and FIG. 34, the purity was 99.3%, and the content of impurity 1' was less than 0.1%.

### Example 8. Preparation of Crystal Form F1

At 25 °C, compound 1 (2 g, purity: 97.5%, with a content of impurity 1' of 2.32%) was added to ethyl acetate (6 V) to obtain mixture 1. Benzenesulfonic acid (0.98 eq) was mixed with ethyl acetate (4 V) to obtain mixture 2. A portion of mixture 2 was added to mixture 1, followed by the addition of the seed crystal of crystal form F1 (such as crystal form F1 of benzenesulfonate prepared in Example 6) (0.1% eq), and the remaining mixture 2 was then added. After the addition was completed, the mixture was stirred for 19 h, cooled to 0 °C, stirred for another 5.5 h, and filtered. The filter cake was washed with ethyl acetate (2 V) and dried at 25 °C for 20 h to obtain crystal form F1 of benzenesulfonate of compound 1 with a purity of 99.5% and a content of impurity 1' of less than 0.1%.

### Efficacy Example 1. Stability Study of Crystal Forms

Three portions of crystal form A1 of benzenesulfonate of compound 1 and crystal form F1 of benzenesulfonate of compound 1 were stored under different conditions for a certain period of time, respectively. The storage conditions and storage time are shown in the table below. The crystal form status of each group was then determined by XRPD, and the results are listed in the table below.

| | Storage condition | Storage time | Original crystal form | Crystal form after treatment |
|---|---|---|---|---|
| 1 | Sealed container at room temperature | 2 months | Crystal form A1 | Crystal form A1 |
| 2 | Sealed container at 2-8 °C | 2 months | Crystal form A1 | Crystal form A1 |
| 3 | Sealed container at 60 °C | 7 days | Crystal form A1 | Crystal form A1 |
| 4 | 25 °C/60% RH, open container | 14 days | Crystal form F1 | Crystal form F1 |
| 5 | Sealed container at 40 °C | 14 days | Crystal form F1 | Crystal form F1 |
| 6 | Sealed container at 2-8 °C | 14 days | Crystal form F1 | Crystal form F1 |
| 7 | Sealed container at 60 °C | 14 days | Crystal form F1 | Crystal form F1 |

The results show that the crystal form has good stability at different temperatures and is not prone to polymorphic transition.

### Efficacy Example 2. Solubility Study of Crystal Forms

An aqueous HCl solution at pH 1 was prepared. 0.6 mL of the solution described above was then added to 8 mg of crystal form F1. After dissolution by stirring, the solution was adjusted to pH = 1 with 1 N NaOH or 1 N HCl. The timing was started, and after T = 3 h, the pH was measured. If the pH showed a significant deviation, the pH was readjusted to the target value. When T = 4 h, the dissolution status was observed, and a dilution factor was selected according to the dissolution status. 50% ACN was used as a diluent for HPLC analysis. When T = 24 h, the dissolution state was observed. With reference to the concentration data at 4 h and the dissolution state at 24 h, a dilution factor was selected, and HPLC analysis was performed. The solubility was calculated according to the HPLC analysis results described above.

The aqueous HCl solution at pH 1 in the step described above was replaced with a 20 mM phosphate buffer solution at pH of about 7, and the steps described above were repeated. The solubility was calculated.

The dissolution status and solubility results are shown in the table below:

| | pH=1 | pH=7 | | |
|---|---|---|---|---|
| Time | 4 h | 24 h | 4 h | 24 h |
| Dissolution status | Clear solution | Clear solution | Clear solution | Clear solution |
| Solubility (mg/mL) | 11.50 | 11.37 | 9.89 | 6.14 |

### Comparative Example 1: Screening of Salt Forms

25 mg of compound 1 prepared in Example 1 was dissolved in 0.5 mL of each solvent, and then an acid or a base (0.55-1.1 eq) was added to the solution at room temperature for a reaction. Various conventional crystallization methods (an anti-solvent method, a solvent volatilization method, a cooling crystallization method, and the like) all failed to induce solid precipitation. The salt formation, crystallization, and impurity removal effects of the products are listed in the table below:

The corresponding situations are as follows:

| Solvent | Acid/base for salt formation | Equivalent | Whether salt can be formed or not | Product form | Impurity removal effect |
|---|---|---|---|---|---|
| MTBE | Benzoic acid | 1.1 | Yes | Oily substance | None |
| Water | Hippuric acid | 1.1 | No | / | None |
| Water | Fumaric acid | 1.1 | No | / | None |
| MTBE | Citric acid | 1.1 | No | / | None |
| Water | Adipic acid | 1.1 | Yes | Oily substance | None |
| Water | Nicotinic acid | 1.1 | Yes | Oily substance | None |
| MTBE | Salicylic acid | 1.1 | Yes | Oily substance | None |
| MTBE | Malic acid | 1.1 | Yes | Oily substance | None |
| MTBE | Diethanolamine | 1.1 | Yes | Oily substance | None |
| IPE/water | Ethylenediamine | 0.55 | Yes | Oily substance | None |
| IPE/water | Calcium hydroxide | 0.55 | Yes | Oily substance | None |
| IPA | Sodium hydroxide | 1.1 | Yes | Oily substance | None |
| MTBE | Aqueous ammonia | 1.1 | Yes | Oily substance | None |
| MTBE ; | Oxalic acid | 1.1 | Yes | Oily substance | None |

| | | | | | |
|---|---|---|---|---|---|
| Note: The "none" in the table means that it has no impurity removal effect. | | | | | |

As can be seen from the above, compound 1 can form salts with some acids or bases, but the physical forms of these salts are oily substances, which cannot achieve the effect of removing the isomer impurity represented by formula 1'.

### Comparative Example 2. Preparation of Other Crystal Forms

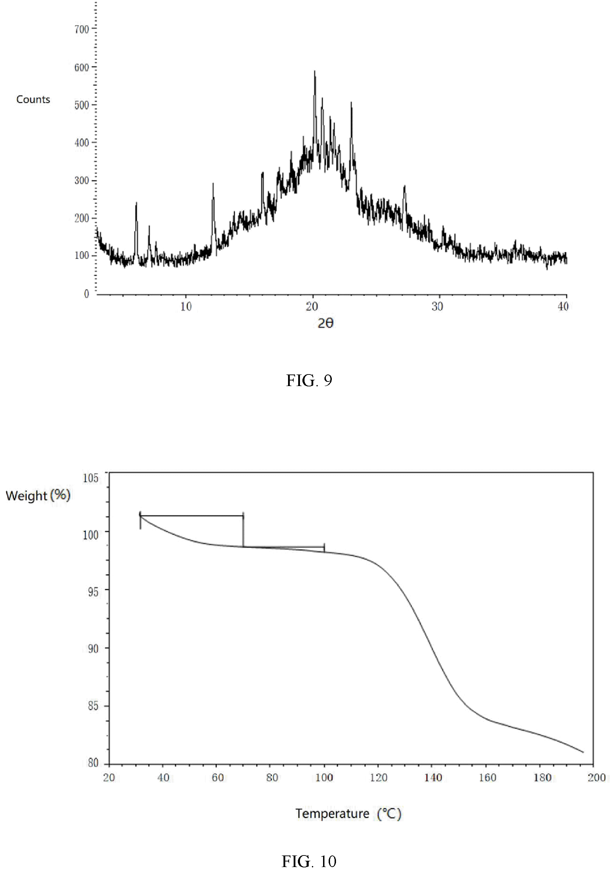

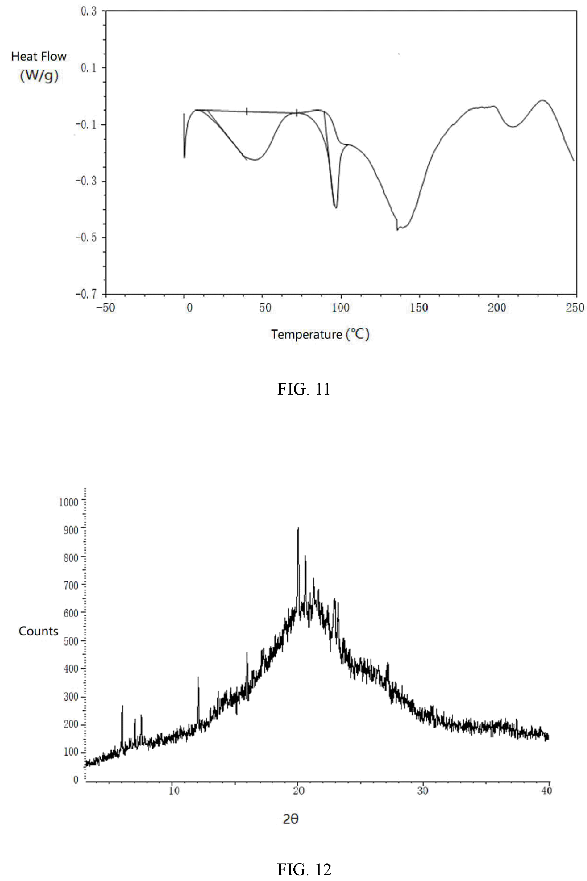

4 mL of ACN was added to 1 g of benzenesulfonate of compound 1, and then the mixture was filtered through a 0.45 µm syringe membrane filter. 32 mL of MTBE was slowly added to the filtrate, and the mixture was stirred at 25 °C for about 4 days. The solid was collected by filtration and characterized by XRPD to obtain 530 mg of crystal form A4 of benzenesulfonate of compound 1, the characterization of which is shown in FIGs. 9-11. However, crystal form A4 rapidly transformed into crystal form F1.

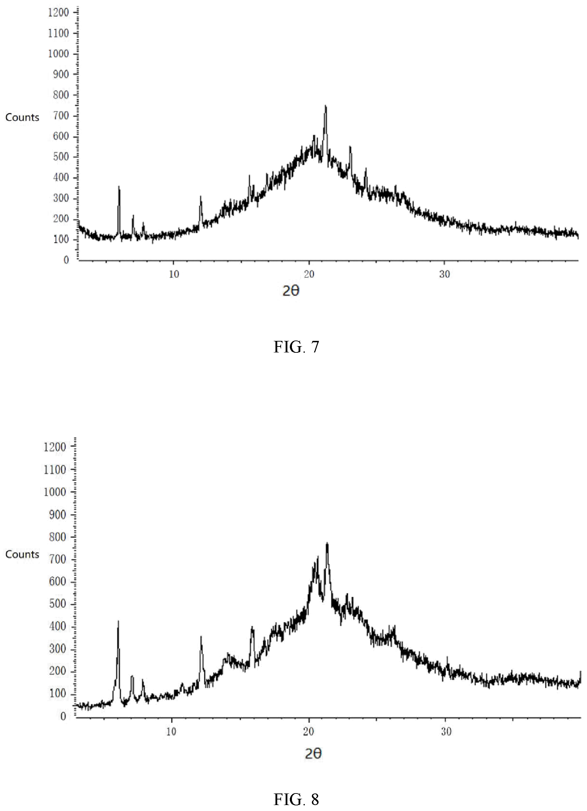

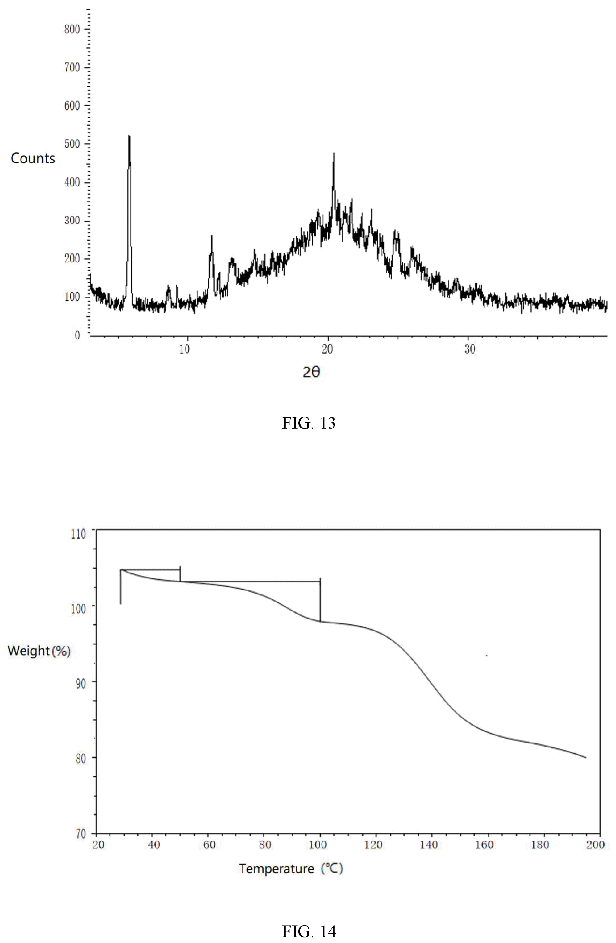

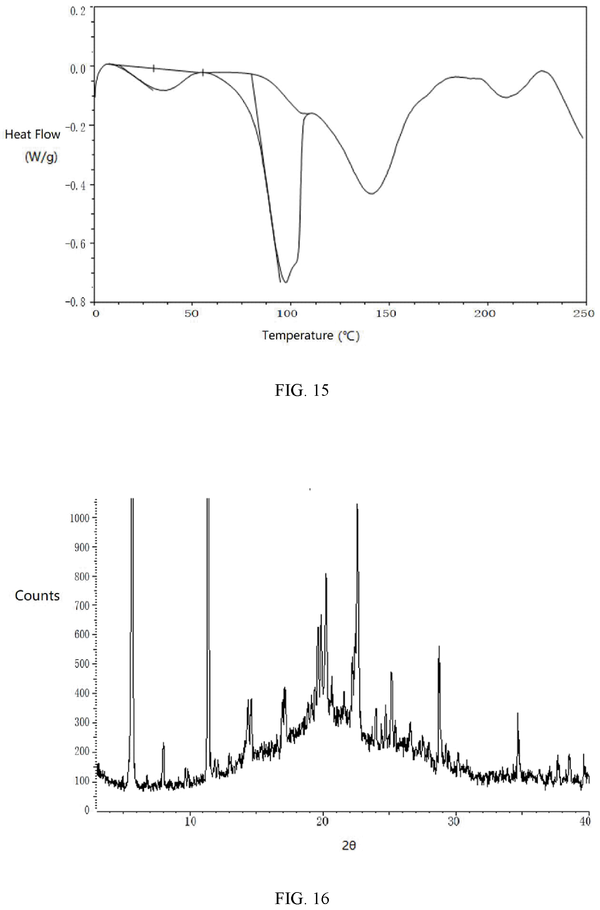

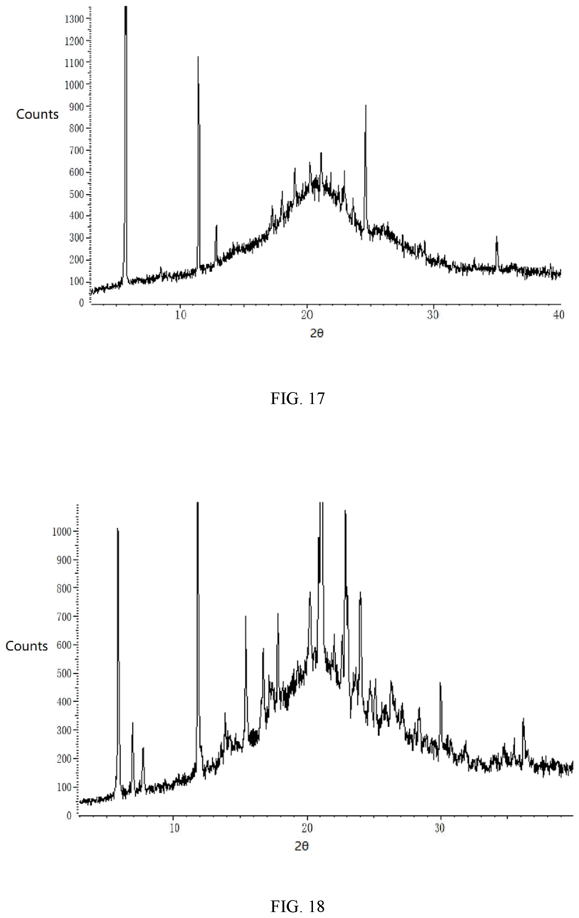

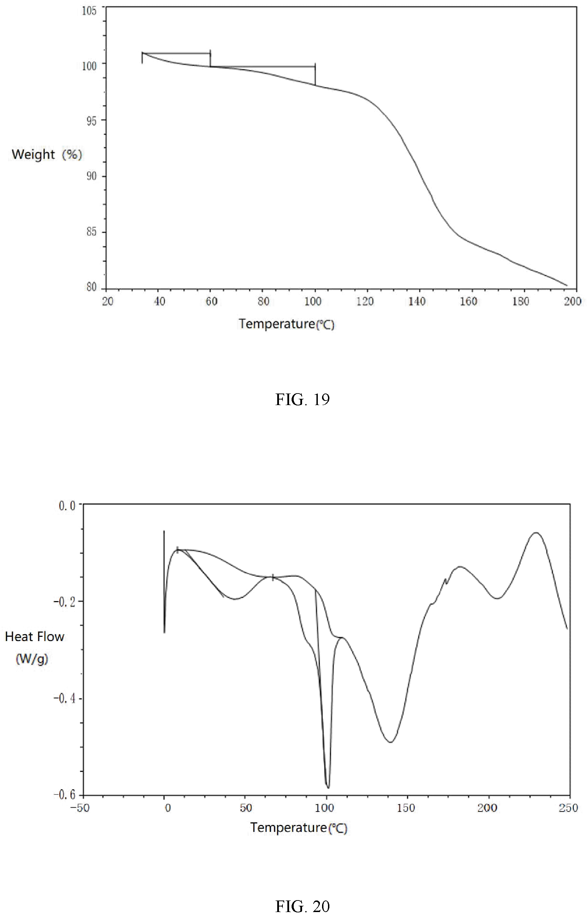

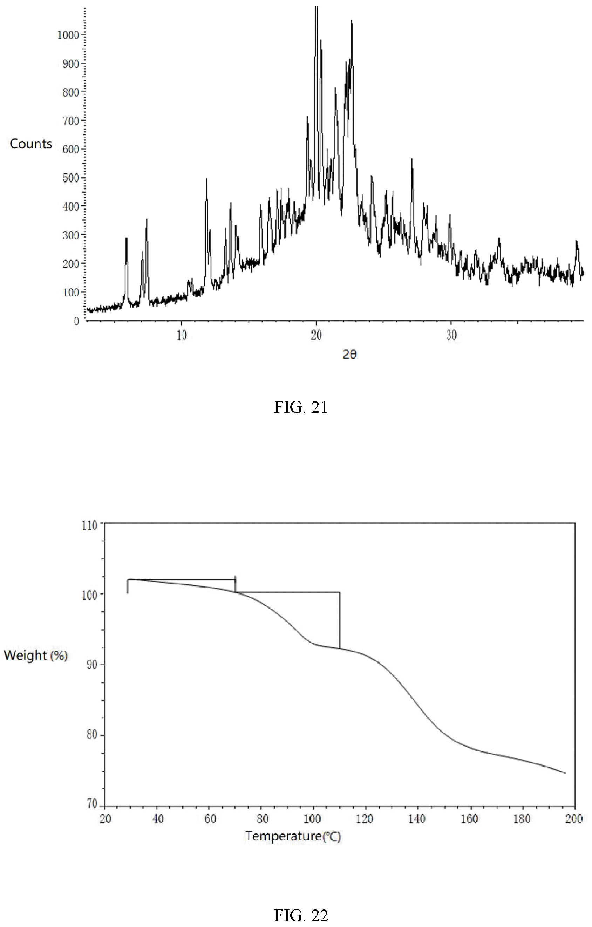

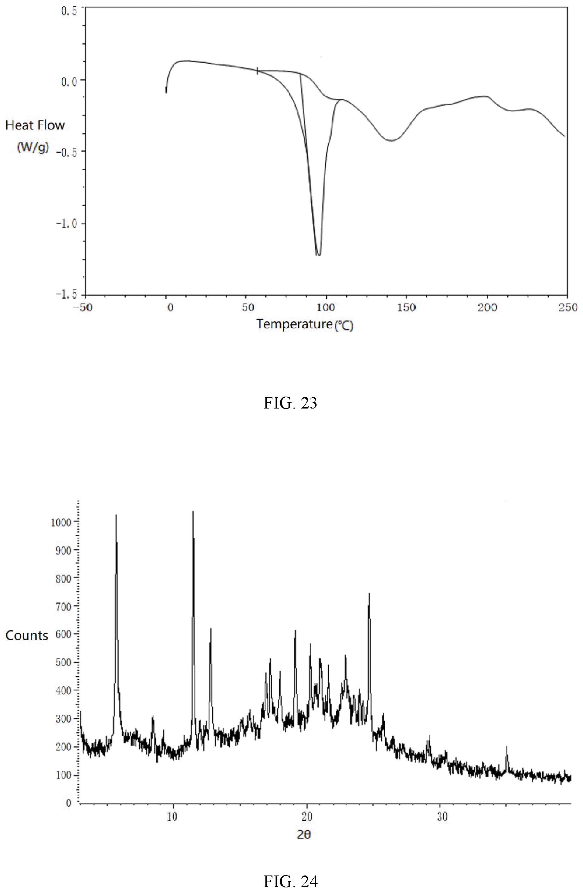

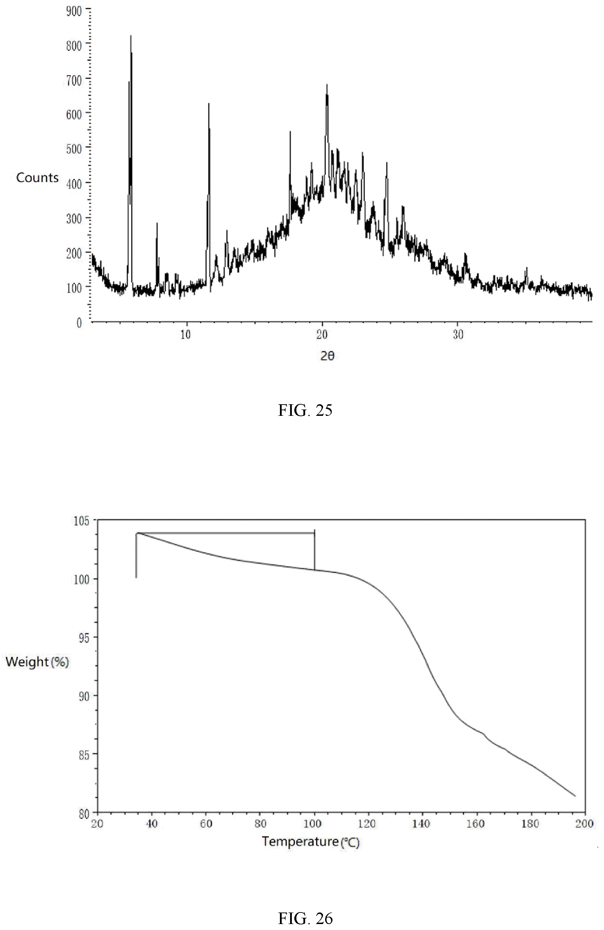

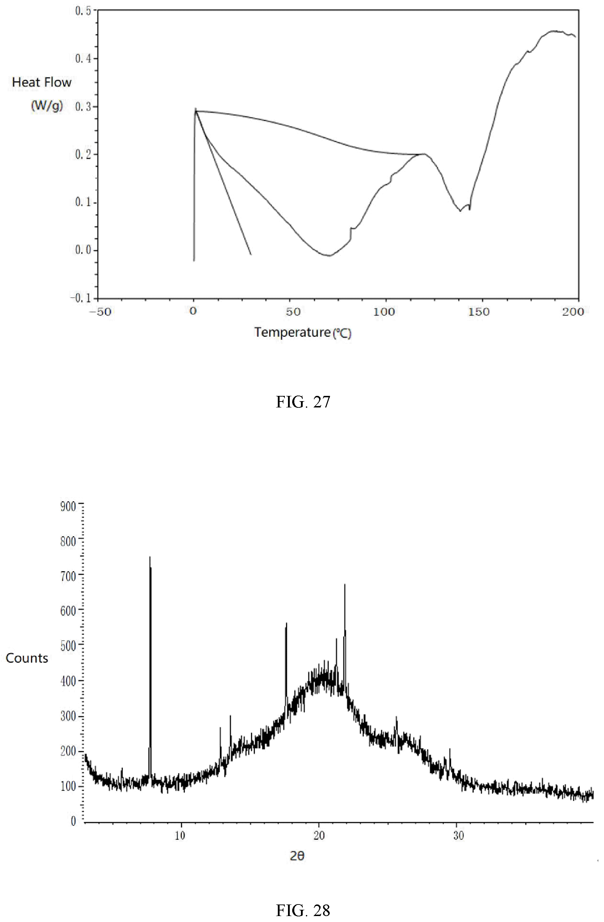

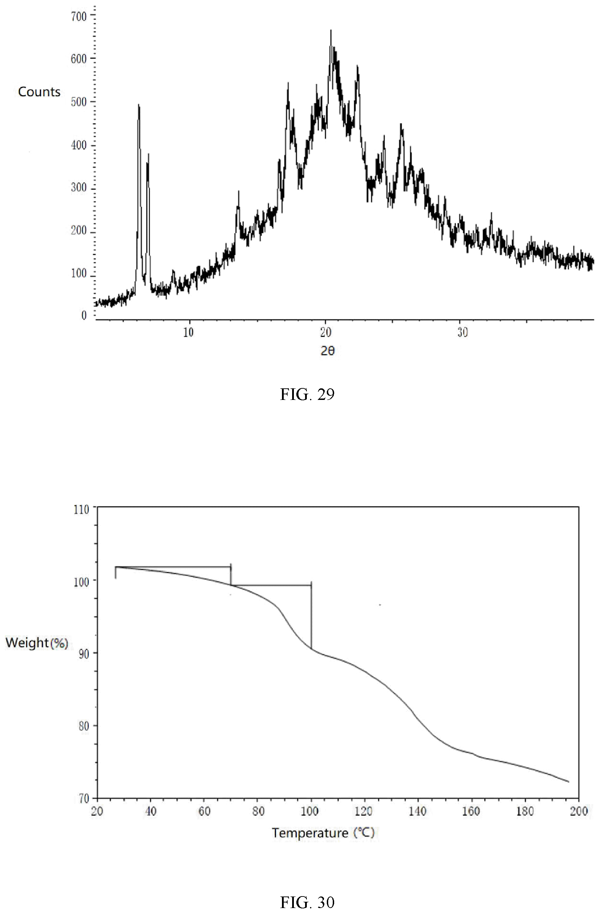

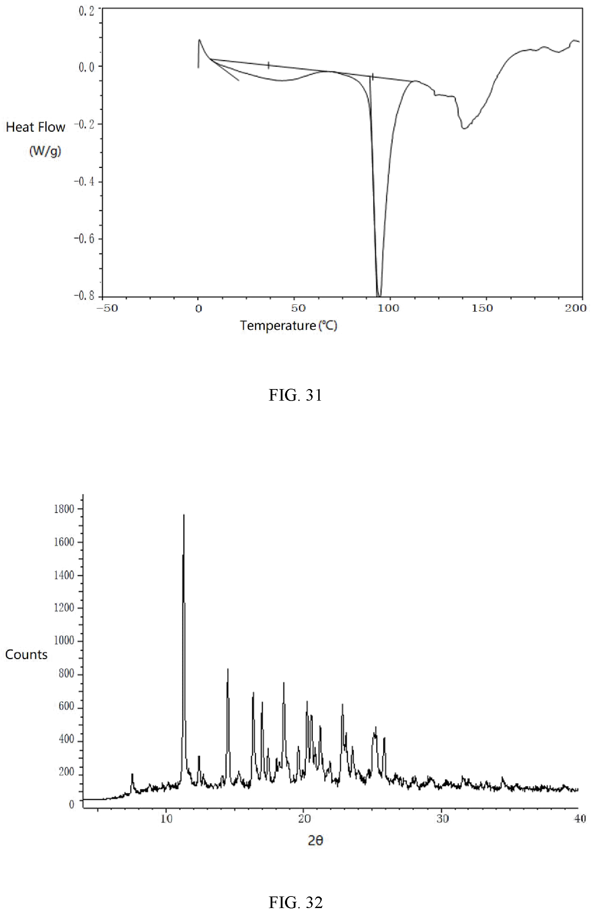

Other crystal forms of compound 2 were also prepared by conventional methods in the present application, and their characterization is shown in FIGs. 7-8 and FIGs. 12-33, while such other crystal forms exhibit poor stability.

## Claims

1. A crystal form A1 of compound 2, wherein, which has diffraction peaks at 2θ angles of 5.971°±0.200°, 7.088°±0.200°, 12.068°±0.200°, 15.881°±0.200°, 20.557°±0.200°, and 21.599°±0.200° in an X-ray powder diffraction pattern using Cu-Kα radiation;

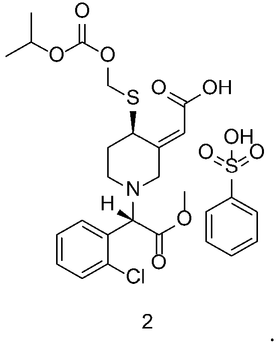

2. The crystal form A1 of compound 2 according to claim 1, wherein one or two of the following conditions are met:
(1) crystal form A1 of compound 2 further has characteristic peaks at one or more 2θ angles of 7.742°±0.200°, 17.257°±0.200°, 21.259°±0.200°, 23.137°±0.200°, and 24.134°±0.200° in an X-ray powder diffraction pattern using Cu-Kα radiation; preferably, diffraction peaks at 2θ angles and relative intensities of crystal form A1 of compound 2 in an X-ray powder diffraction pattern using Cu-Kα radiation are:
| 2θ angle | Relative intensity |
|---|---|
| 5.971° | 100.0% |
| 7.088° | 61.3% |
| 7.742° | 23.7% |
| 10.667° | 5.7% |
| 12.068° | 41.9% |
| 12.357° | 4.7% |
| 13.727° | 6.5% |
| 14.076° | 19.7% |
| 15.881° | 58.6% |
| 17.257° | 22.7% |
| 17.803° | 5.2% |
| 18.184° | 7.0% |
| 18.693° | 4.4% |
| 19.379° | 8.7% |
| 20.225° | 10.8% |
| 20.557° | 42.0% |
| 21.259° | 35.0% |
| 21.599° | 54.1% |
| 22.746° | 18.0% |
| 23.137° | 30.7% |
| 23.657° | 10.3% |
| 24.134° | 29.5% |
| 25.032° | 7.2% |
| 26.215° | 16.0% |
| 27.112° | 2.3% |
| 27.630° | 3.5% |
| 28.106° | 6.2% |
| 29.093° | 4.6% |
| 29.430° | 2.6% |
| 30.103° | 3.8% |
| 32.135° | 2.5% |
| 36.787° | 2.2% |
further, crystal form A1 of compound 2 has an X-ray powder diffraction pattern, expressed in terms of 2θ angles, using Cu-Kα radiation substantially as shown in FIG. 1;
(2) a thermogravimetric analysis (TGA) pattern of crystal form A1 of compound 2 shows a weight loss of 3.63% at 27-100 °C, wherein "%" is mass percentage; further, crystal form A1 of compound 2 has a TGA pattern substantially as shown in FIG. 3.

3. A crystal form F1 of compound 2, wherein, which has diffraction peaks at 2θ angles of 17.006°±0.200°, 20.439°±0.200°, 22.149°±0.200°, and 23.629°±0.200° in an X-ray powder diffraction pattern using Cu-Kα radiation;

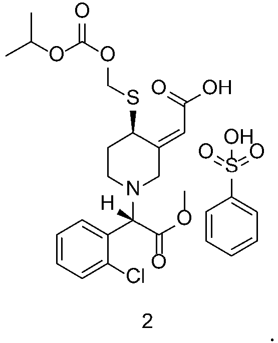

4. The crystal form F1 of compound 2 according to claim 3, wherein one or more of the following conditions are met:
(1) the crystal form F1 of compound 2 further has characteristic peaks at one or more 2θ angles of 17.891°±0.200°, 19.671°±0.200°, 27.740°±0.200°, and 28.842°±0.200° in an X-ray powder diffraction pattern using Cu-Kα radiation; preferably, diffraction peaks at 2θ angles and relative intensities of crystal form A1 of compound 2 in an X-ray powder diffraction pattern using Cu-Kα radiation are:
| 2θ angle | Relative intensity |
|---|---|
| 7.978° | 16.10% |
| 10.514° | 3.30% |
| 11.115° | 1.30% |
| 12.164° | 8.90% |
| 13.115° | 11.30% |
| 13.834° | 15.10% |
| 15.404° | 1.50% |
| 16.063° | 29.60% |
| 16.254° | 3.20% |
| 17.006° | 100.00% |
| 17.891° | 41.10% |
| 18.146° | 31.30% |
| 18.617° | 16.70% |
| 19.671° | 34.90% |
| 20.439° | 54.60% |
| 21.186° | 8.80% |
| 21.551° | 29.30% |
| 22.149° | 82.50% |
| 22.741° | 19.70% |
| 23.036° | 7.00% |
| 23.629° | 84.80% |
| 24.692° | 25.60% |
| 25.104° | 30.40% |
| 25.522° | 17.20% |
| 25.915° | 34.10% |
| 26.888° | 14.10% |
| 27.740° | 50.10% |
| 28.118° | 15.30% |
| 28.488° | 8.00% |
| 28.842° | 43.60% |
| 29.426° | 13.80% |
| 29.803° | 22.60% |
| 30.588° | 25.80% |
| 31.022° | 18.40% |
| 31.227° | 12.60% |
| 31.772° | 12.00% |
| 32.153° | 4.40% |
| 32.802° | 11.70% |
| 33.117° | 9.80% |
| 33.785° | 5.60% |
| 34.061° | 6.20% |
| 34.582° | 6.80% |
| 35.040° | 6.40% |
| 35.525° | 5.20% |
| 35.838° | 4.60% |
| 36.460° | 11.30% |
| 37.166° | 13.40% |
| 37.905° | 6.80% |
| 38.351° | 8.30% |
| 38.639° | 9.80% |
| 39.699° | 4.20% |
further, crystal form F1 of compound 2 has an X-ray powder diffraction pattern, expressed in terms of 2θ angles, using Cu-Kα radiation substantially as shown in FIG. 4;
(2) the thermogravimetric analysis (TGA) pattern of crystal form F1 of compound 2 shows a weight loss of 0.5% at 32-130 °C, "%" is mass percentage; further, crystal form F1 of compound 2 has a TGA pattern substantially as shown in FIG. 5; and
(3) a differential scanning calorimetry (DSC) pattern of crystal form F1 of compound 2 shows an endothermic peak at 147.8 °C; further, crystal form F1 of compound 2 has a DSC pattern substantially as shown in FIG. 6.

5. A preparation method for the crystal form A1 of compound 2 according to any one of claims 1-2, wherein, comprising the following step: crystallizing compound 2 in a solvent, or mixing compound 1 with benzenesulfonic acid in a solvent, the solvent is a mixed solvent of an ether solvent and acetonitrile; further, the preparation method comprises the following step: mixing solution 1 containing compound 1 with solution 2 containing benzenesulfonic acid for a reaction, wherein a solvent of solution 1 is a mixed solvent of an ether solvent and acetonitrile; a solvent of solution 2 is a mixed solvent of an ether solvent and/or acetonitrile; the ether solvent may be isopropyl ether;

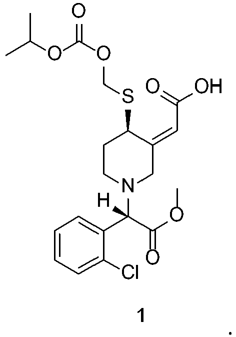

6. The preparation method for the crystal form A1 of compound 2 according to claim 5, wherein one or more of the following conditions are met:
(1) a purity of compound 1 is 90%-99.6%, such as 92.6%, 94.3%, 95.8%, or 99%;
(2) compound 1 is a crude product of compound 1, and the crude product of compound 1 is a mixture of compound 1 and an isomer impurity represented by formula 1'; a mass ratio of compound 1 to the isomer impurity may be (10-500):1, such as 15.433:1, 23.575:1, 23.95:1, or 220:1; 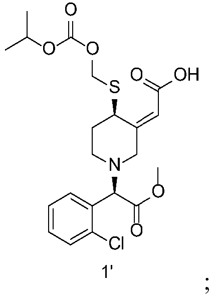
(3) when the solvent is an ether solvent and a nitrile solvent, a volume ratio of the ether solvent to the nitrile solvent is (1-10):1, such as 5:1 or 7:1;
(4) a mass-to-volume ratio of compound 1 to the solvent of solution 1 is 1:(1-15) g/mL, or may be 1:(1-10) g/mL, such as 1:6 g/mL and 1:10 g/mL;
(5) a molar ratio of benzenesulfonic acid to compound 1 is (0.9-1.2):1, such as 1.1:1;
(6) a mass-to-volume ratio of benzenesulfonic acid to the solvent of solution 2 is 1:(0.1-10) g/mL, such as 1:6 g/mL or 1:10 g/mL;
(7) the preparation method for crystal form A1 of compound 2 further comprises a step of mixing with crystal form A1 of compound 2 according to any one of claims 1-2 as a benzenesulfonate seed crystal; a mass ratio of the benzenesulfonate seed crystal to compound 1 may be 1:(20-100), such as 1:50;
(8) the preparation method for crystal form A1 of compound 2 further comprise a step of mixing with solution 3 containing crystal form A1 of compound 2 according to any one of claims 1-2 as a benzenesulfonate seed crystal, wherein a solvent of solution 3 is a mixed solvent of an ether solvent and/or acetonitrile; the ether solvent may be isopropyl ether;
(9) the preparation method for crystal form A1 of compound 2 further mix with solution 3 containing crystal form A1 of compound 2 according to any one of claims 1-2 as a benzenesulfonate seed crystal, a mass-to-volume ratio of the benzenesulfonate seed crystal to the solvent of solution 3 is 1:(6000-10000) g/mL, such as 1:8000 g/mL;
(10) a mixing temperature is 25 °C; and
(11) a mixing reaction time is 0.1-100 h, such as 3 h, 17 h, or 72 h.

7. A preparation method for crystal form F1 of compound 2 according to any one of claims 3-4, wherein, comprising the following step: crystallizing compound 2 in a solvent, or mixing compound 1 with benzenesulfonic acid in a solvent, the solvent is an ester solvent; further, the preparation method comprises the following step: mixing solution 1 containing compound 1 with solution 2 containing benzenesulfonic acid, wherein a solvent of solution 1 is an ester solvent or an ether solvent; a solvent of solution 2 is an ester solvent; the ester solvent may be ethyl acetate and/or isopropyl acetate; the ether solvent may be tetrahydrofuran;

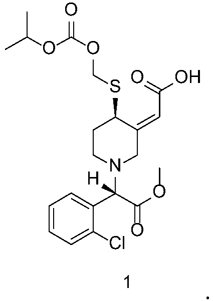

8. The preparation method for the crystal form F1 of compound 2 according to claim 7, wherein one or more of the following conditions are met:
(1) a purity of compound 1 is 90%-99.6%, such as 95.8%, 97.14%, or 97.5%;
(2) compound 1 is a crude product of compound 1, and the crude product of compound 1 is a mixture of compound 1 and an isomer impurity represented by formula 1'; a mass ratio of compound 1 to the isomer impurity may be (10-500):1, such as 23.95:1, 33.965:1, or 42.026:1; 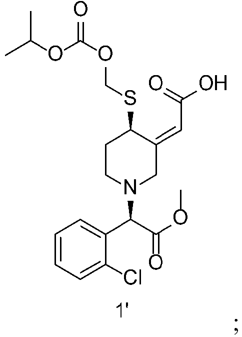
(3) a mass-to-volume ratio of compound 1 to the solvent of solution 1 is 1:(1-15) g/mL, or may be 1:(1-10) g/mL, such as 1:2.3 g/mL, 1:4 g/mL, or 1:6 g/mL;
(4) a molar ratio of benzenesulfonic acid to compound 1 is (0.9-1.2):1, such as 0.98:1 or 1:1;
(5) a mass-to-volume ratio of benzenesulfonic acid to the solvent of solution 2 is 1:(0.1-10) g/mL, such as 1:0.7 g/mL, 1:1 g/mL, or 1:4 g/mL;
(6) the preparation method for crystal form F1 of compound 2 further comprises a step of mixing with solvent 3, solvent 3 is an alkane solvent, and the alkane solvent may be n-heptane; a mass-to-volume ratio of compound 1 and solvent 3 is 1:(3-45) g/mL, such as 1:27 g/mL;
(7) the preparation method for crystal form F1 of compound 2 further comprises thermal cycling; number of thermal cycles may be 5; and
(8) the preparation method for crystal form F1 of compound 2 further comprises thermal cycling; thermal cycling temperature may be 25 °C to -10 °C.

9. Use of crystal form A1 of compound 2 accoriding to any one of claims 1-2 or crystal form F1 of compound 2 accoriding to any one of claims 3-4 in the preparation of compound 1;

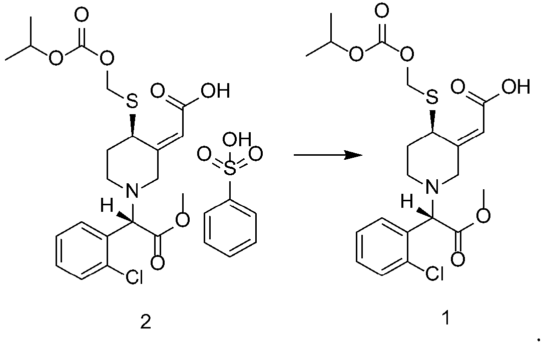

10. A purification method for compound 1, wherein the purification method comprises the following steps: 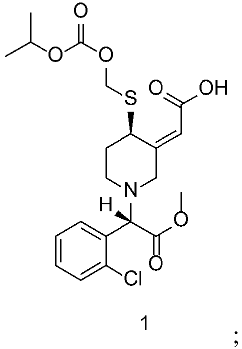
Scheme I:
S1: the preparation method for crystal form A1 of compound 2 accoriding to claim 5 or 6;
S2: subjecting crystal form A1 of compound 2 to a hydrolysis reaction to prepare compound 1;
Scheme II:
S1: the preparation method for crystal form F1 of compound 2 accoriding to claim 7 or 8 ;
S2: subjecting crystal form F1 of compound 2 to a hydrolysis reaction to prepare compound 1.
